Europäisches Patentamt
(19) European Patent Office
Office européen des brevets

(11) Publication number: 0 011 828 A1

# (12) EUROPEAN PATENT APPLICATION

(21) Application number: 79104650.1

(22) Date of filing: 22.11.79

(51) Int. Cl.3: A 61 K 45/06
A 61 K 31/52, A 61 K 31/505
//(A61K31/52, 31/415),
(A61K31/505, 31/415)

(30) Priority: 23.11.78 GB 4584678

(43) Date of publication of application:
11.06.80 Bulletin 80/12

(84) Designated Contracting States:
BE CH DE FR GB IT NL SE

(71) Applicant: THE WELLCOME FOUNDATION LIMITED
183-193 Euston Road
London NW1 2BP(GB)

(72) Inventor: Moncada, Salvador Enrique
17 St. Davids Close
West Wickham, Kent(GB)

(74) Representative: Berg, Wilhelm, Dr. et al,
Dr. Berg, Dipl.-Ing. Stapf, Dipl.-Ing. Schwabe, Dr. Dr.
Sandmair Mauerkircherstrasse 45
D-8000 München 80(DE)

## (54) Pharmaceutical combinations including hydantoin derivatives, pharmaceutical formulations containing the combinations and methods of making the formulations.

(57) Combinations of an active compound selected from the class outstanding of hydantoin and thiohydantoin derivatives and their salts together with a phosphodiesterase inhibitor. Such combinations have pharmacological effects related to those of the active compound alone but these effects (other than vasodilatory effects) are potentiated by the phosphodiesterase inhibitor whilst the vasodilatory effect is not so potentiated. The combination may be used in medicine either along or in a formulation, for example in the treatment of thrombosis.

EP 0 011 828 A1

Croydon Printing Company Ltd.

DIPL.-ING. SCHWABE DR. DR. SANDMAIR
PATENTANWÄLTE
Postfach 86 02 45 · 8000 München 86

0011828

Attorney's file: 50 055 22nd November 1979

THE WELLCOME FOUNDATION LIMITED

London / Great Britain

---

Pharmaceutical combinations including hydantoin derivates, pharmaceutical formulations containing the combinations and methods of making the formulations

---

The present invention relates to the use in medicine of hydantoin and thiohydantoin derivatives or their salts in conjunction with a phosphodiesterase inhibitor and to a pharmaceutical formulation containing them.

☎ (089) 98 82 72
98 82 73
98 82 74
98 33 10

Telegramme:
BERGSTAPFPATENT München
TELEX:
05 24 560 BERG d

Bankkonten: Hypo-Bank München 4410122850
(BLZ 70020011) Swift Code: HYPO DE MM
Bayer Vereinsbank München 453100 (BLZ 70020270)
Postscheck München 65343-808 (BLZ 70010080)

Some compounds selected from hydantoin and thiohydantoin derivatives and their salts have been disclosed as having a variety of pharmacological effects including a potent anti-aggregatory action on blood platelets. They are therefore of value as therapeutic agents in the treatment or prevention of thrombo-embolic disorders in mammals.

Other pharmacological effects exhibited by such hydantoin and thiohydantoin derivatives and their salts include inhibition of pentagastrin-induced gastric acid secretion; diuretic properties; effects on the uterine smooth muscle; effects on the biochemical co-operation between platelets and vascular endothelium, which contribute to the repair of damaged vascular endothelium; and antagonism of histamine-induced broncho-constriction.

In addition, many such compounds also exhibit a vasodilatory action on blood vessels.

Hydantoin and thiohydantoin derivatives and their salts which exhibit both vasodilatory action <u>and</u> another pharmacological effect

as described hereinbefore are referred to hereinafter as the 'active compounds'.

There are often occasions when it would be desirable to be able to utilize the pharmacological effects referred to above, notably the anti-aggregatory effect, while at the same time suppressing or eliminating the vasodilatory effect of the active compounds; for example in the treatment or prevention of myocardial infarcts and when they are used as an addition in extra-corporeal circulation.

We have now surprisingly discovered that the pharmacological effects, but not the vasodilatory effects of the active compounds, are potentiated by chemical compounds which are cyclic AMP phosphodiesterase inhibitors. This discovery provides a means to dissociate the anti-aggregatory and other pharmacological effects from the vasodilatory effect of the active compounds.

Accordingly the present invention provides a combination which comprises:

(a) an active compound and

(b) a phosphodiesterase inhibitor;

which is hereinafter referred to as 'the combination'.

The combination lowers the threshold level of the pharmacological effect (not including vasodilatory effects) (the minimum amount of active compound required to exhibit a certain pharmacological effect) without substantially affecting the vasodilatory threshold level. Thus for

example by administering an active compound (which exhibits an anti-aggregatory effect) at a concentration below its vasodilatory threshold level, but above the new, lower, anti-aggregatory threshold, together with a phosphodiesterase inhibitor, anti-aggregatory effects can be produced whilst vasodilatory effects are not.

Active compounds which exhibit a variety of the pharmacological effects referred to above include 1,5-disubstituted hydantoin and thiohydantoin derivatives optionally further substituted in the 3- and/or the 5-position of the hydantoin ring. Examples of such active compounds and their preparation may be found in the following patent specifications:
Belgian Patent No 855 337, Japanese Nos 63083/79 and 95571/79, Eire patent application No 2370/78, and Belgian Patent No 861 956 and European patent publication No 3-410 (European application No 79 30008.4).

Active compounds which exhibit interesting vasodilatory and other pharmacological effects include those of formula (I)

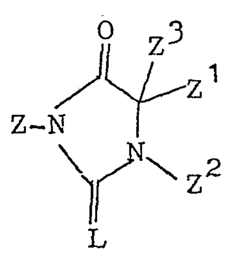

(I)

wherein

L is O or S;

Z and $Z^3$ are the same or different and each is hydrogen or alkyl of 1 to 6 carbon atoms;

one of $Z^1$ and $Z^2$ is a group $-CH_2-X-X^1-X^2$ wherein

X is phenylene, -C≡C-, cis or trans -CH=CH- or $-CH_2-CQ_2-$ in which each Q is independently selected from hydrogen and alkyl such as ethyl or the two Q's together form an alkylene radical of four, five or six carbon atoms;

$X^1$ is a covalent bond or a straight or branched alkylene chain having 1 to 6 carbon atoms optionally having one of its methylene groups replaced by oxa (-O-) or thia (-S-) provided that at least one carbon atom separates any oxa or thia from a -C≡C-, -CH=CH- or -CO- group; and

$X^2$ is selected from 5-tetrazolyl, carboxyl, carboxamide, hydroxymethylene and alkoxycarbonyl;

and the other of $Z^1$ and $Z^2$ is a group $-Y-Y^1-Y^2-Y^3$ wherein

Y is $-CR_2-CH_2-$ in which each R is independently selected from hydrogen and methyl;

$Y^1$ is carbonyl, methylene, methylene substituted by hydroxyl or methylene substituted by hydroxyl and alkyl;

$Y^2$ is a covalent bond or straight or branched alkylene having 1 to 7 carbon atoms optionally substituted in the carbon adjacent $Y^1$ by one or two groups each of which may be alkyl or a cyclic radical;

$Y^3$ is hydrogen, hydroxy, alkoxy of 1 to 7, preferably 1 to 4, carbon atoms, a cyclic radical, phenyl, benzyl, phenoxy or benzyloxy, wherein each of phenyl, benzyl, phenoxy and benzyloxy may be substitued in the benzene ring by one or more groups selected from hydroxy, halogeno, nitro, amino, acylamino, alkenyl, alkoxy, phenyl and alkyl

which may itself be substituted by one or more halogeno groups; or $Y^2$ and $Y^3$ together form an alkyl group of 1 to 7 carbon atoms having at least one hydrogen replaced by fluoro;

or Y is a bond, $-CH_2-$ or $-CH_2.CH_2-$ and $Y^1$, $Y^2$ and $Y^3$ taken together form a cycloalkyl or bicycloalkyl group substituted by a hydroxyl group which preferably has three carbon atoms separating it from the hydantoin ring.

In formula (I), the term cyclic radical means the monovalent radical derived by loss of a ring hydrogen atom from a monocyclic or polycyclic compound having from 3 to 12 ring atoms selected from carbon, nitrogen, oxygen, and sulphur, which compound may be saturated or unsaturated and may be further substituted by one or more alkyl groups, but excluding phenyl. Such cyclic radicals include cycloalkyl having 3 to 10 carbon atoms such as cyclopropyl, cyclopentyl, cyclohexyl and cyclooctyl; bicycloalkyl having 4 to 10 carbon atoms such as norbornyl (bicyclo 2,2,1 heptyl), spiroalkanyl having 5 to 12 carbon atoms such as 2-spiro 3,3 heptyl, 1-spiro 4,4 nonane and 8-spiro 4,5 decane, or adamantyl; cycloalkenyl having 4 to 10 carbon atoms such as 4-cyclopentene; heterocyclic radicals such as tetrahydrofuranyl and tetrahydropyranyl and heteroaryl radicals such as thienyl, furyl, pyridyl, pyrimidyl, thiazolyl, imidazolyl and diazapinyl. Included in the term cyclic radical are these wherein one or more hydrogen atoms are replaced by fluoro.

JDM/LM/18 October 1979

Unless otherwise stated, in formula (I) and other formulae in this specification, alkyl moieties are selected from methyl, ethyl, propyl, butyl, pentyl and hexyl, including all isomers thereof; for example, in the definitions of $Y^1$, $Y^2$ and Z the alkyl groups are preferably methyl; and the alkyl moiety of alkoxycarbonyl is desirably methyl or ethyl. Alkenylene groups have 2 to 4 carbon atoms, for example vinyl.

In a compound of formula (I) the bonding of the divalent phenylene group may be ortho, meta or para, and the oxa or thia group is preferably adjacent to the phenylene or when X is other than phenylene then $X^1$ may be $-CH_2-O-CH_2$ or $-CH_2-S-CH_2-$.

Included in the meaning of compound of formula (I) are the salts corresponding to the carboxylic acids and tetrazoles when $X^2$ is carboxyl or tetrazolyl respectively, anf the salts which may also be formed when Z is hydrogen. Particularly valuable salts for medical purposes are those having a pharmaceutically acceptable cation such as ammonium or that of an alkali metal e.g. sodium and potassium, an alkaline earth metal e.g. calcium and magnesium, or an organic base, particularly an amine such as ethanolamine. Salts having non-pharmaceutically acceptable cations are included within the ambit of this invention as useful intermediates to pharmaceutically acceptable salts, or the acids or esters of formula (I).

Except when there is clear indication to the contrary, formula (I) and other formulae in the specification embrace all stereoisomers represented therein. In particular such formulae include the enantiomeric forms, such mixtures as are designated racemates, and diastereoisomers.

Active compounds of the formula (I) which exhibit especially interesting anti-aggregatory and vasodilatory action are those where in formula (I)

Z is hydrogen or alkyl having 1 to 4 carbon atoms, for example methyl or butyl; $Z^3$ is hydrogen;

one of $Z^1$ and $Z^2$ is $-CH_2-X-X^1-X^2$ wherein X is $-CH_2.CH_2-$ or $-CH=CH-$; $X^1$ is alkylene of ~~1~~ to 5 in particular 3 carbon atoms, and $X^2$ is alkoxy-carbonyl, carboxyl or a salt thereof;

and the other of $Z^1$ and $Z^2$ is $-Y-Y^1-Y^2-Y^3$ wherein Y, $Y^1$ and $Y^2$ are as hereinbefore defined and $Y^3$ is hydrogen, phenyl, benzyl, or cycloalkyl of 4 to 7 carbon atoms.

A group of active compounds which have been found more especially valuable as inhibitors of platelet aggregation are those of formula (I) wherein Z and $Z^3$ are both hydrogen; $Z^1$ is carboxyalkylene or carboxyalkenylene wherein the alkylene or alkenylene moiety has 3 to 9 carbon atoms; and $Z^2$ is a group $-(CH_2)_2.CH(OH).Y^2.Y^3$ wherein $Y^2$ is branched alkylene having a tertiary carbon atom adjacent to the hydroxy substituted carbon and $Y^3$ is as defined in formula (I).

Within this group of active compounds, those wherein $Z^1$ is carboxyhexyl or carboxyhexenyl and $Y^3$ is cycloalkyl having 4 to 7 carbon atoms have been found especially active; for example, 5-(6-carboxyhexyl)-1-(3-hydroxy-3-cyclohexylpropyl)hydantoin and 5-(6-carboxyhex-2-enyl)-1-(3-hydroxy-3-cyclohexylpropyl)hydantoin.

Suitable phosphodiesterase inhibitors for use in potentiating the anti-aggregatory effects of the active compounds include:-

Xanthine derivatives such as:

Theophylline (3,7-dihydro-1,3-dimethyl-1_H_-purine-2,6-dione) , and salts thereof.

3-Isobutyl-1-methyl-xanthine;

Caffeine (3,7-dihydro-1,3,7-trimethyl-1_H_-purine-2,6-dione) and salts thereof; and

Aminophylline (adduct of Theophylline and 1,2-ethanediamine (2:1)).

Phosphodiesterase inhibitors other than xanthine derivatives that may be used in the combinations of the present invention include, as such or as pharmaceutically acceptable salts:

(a) Isoquinoline derivatives, for example:

Papaverine (1-[(3,4-dimethoxyphenyl)methyl]-6,7-dimethoxyisoquinoline), and salts thereof; and

6,7-Diethoxy-1-(4,5-diethoxybenzyl)isoquinoline, or its salts e.g. its hydrochloride;

(b) Derivatives of pyrimido (5,4-d)pyrimidine, for example:

Dipyridamole (2,2', 2",2'"-(4,8-dipiperidinopyrimido [5,4-d] pyrimidine-2,6-diyldinitrilo)tetraethanol) and its salts;

2,2',2",2"',-[[4-(1-piperidinyl)pyrimido [5,4-d] pyrimidin-2,6-diyl]dinitrilo] tetrakisethanol and its salts; and

2,4,6-tri-4-morpholinylpyrimido [5,4-d] pyrimidine and its salts;

(c) Derivatives of thieno [3,2-d] pyrimidine, for example:

N-[4-(4-morpholinyl)thieno[3,2-d]pyrimidin-2-yl]-1,2-ethanediamine;

(d) Derivatives of pyrazolo[3',4':2,3]pyrido-[4,5-b] [1,5] benzodiazepin-6-(3H)-one, for example:

3-Ethyl-7,12-dihydro-7, 12-dimethylpyrazolo-[4',3':5,6]pyrido[4,3-b] - [1,5]benzodiazepin-6-(3H)-one;

3-Ethyl-7,12-dihydro-9-methoxy-7, 12-dimethyl-pyrazolo[3',4':2,3]pyrido [4,5-b][1,5] benzodiazepin-6-(3H)- one; and

10-Chloro-3-ethyl-7, 12-dimethyl-7, 12-dihydro pyrazolo [4',3':5,6]pyrido [4,3-b][1,5]benzodiazepin-6-(3H)-one;

(e) Derivatives of 1H- or 2H-pyrazolo[3,4-b] pyridine, for example:

4-(Butylamino)-1-ethyl-1H-pyrazolo [3,4-b] pyridine-5-carboxylic acid ethyl ester;

4-(Butylamino)-1H-pyrazolo [3,4-b] pyridine-6-carboxylic acid ethyl ester;

4-Chloro-1-ethyl-3-methyl-1H-pyrazolo[3,4-b]-pyrimidine-5-acetonitrile;

1-Ethyl-4-(isopropylidenehydrazino)-3-methyl-1H-pyrazolo[3,4-b]pyridine-5-carboxylic acid ethyl ester or its salts such as its hydrochloride hemihydrate; and

2-Methyl-6-phenyl-4-(1-piperidinyl)-2H-pyrazolo [3,4-b] pyridine or its salts e.g. its hydrochloride;

(f) Derivatives of 5H-furo-[3,4-e] pyrazolo [3,4-b] pyridine-5-one, for example:

4-(Butylamino)-1-ethyl-1, 7-dihydro-7-hydroxy-5H-furo-[3,4-e] pyrazolo [3,4-b] pyridine-5-one; and

(g) Derivatives of 1-(2H)-naphthalenone, for example:

2 [(Dimethylamino)methyl]-3,4-dihydro-7-methoxy-1-(2H)naphthalenone or its salts e.g. its 1:1 hydrochloride.

A particularly preferred combination is one comprising the less polar isomer of 5-(6-carboxyhexyl)-1-(3-hydroxy -3-cyclohexylpropyl) hydantoin m.p. 96-98$^{\circ}$ as active compound and dipyridamole as phosphodiesterase inhibitor.

In this combination the dipyridamole has produced a potentiation of the anti-aggregatory effect of the active compound in aggregation tests on rabbits and in human platelet rich plasma.

Another particularly preferred combination is one comprising the less polar isomer of 5-(6-carboxyhex-2-enyl)-1-(3-hydroxy-3-cyclohexylpropyl)hydantoin m.p. 97-99$^{\circ}$ as active compound and dipyridamole or theophylline as phosphodiesterase inhibitor.

The combinations are of value as therapeutic agents for use in conditions where any of the pharmacological

effects mentioned hereinbefore are required, other than vasodilation.

For example, because of the anti-aggregatory action of an active compound, the combination may be used to treat or prevent thrombo-embolic disorders e.g. the formation of thrombi in mammals, including man. For example, the combinations are useful in the treatment and prevention of myocardial infarcts, to treat and prevent thrombosis, to promote patency of vascular grafts following surgery, and to treat complications of arteriosclerosis and conditions such as atherosclerosis, blood clotting defects due to lipidemia, and other clinical conditions in which the underlying aetiology is associated with lipid imbalance or hyperlipidemia.

The active compounds, and therefore the combinations containing them, are especially useful as additives to blood, blood products, blood substitutes and other fluids which are used in artificial extra-corporeal circulation and perfusion of an isolated body portion, e.g. a limb or an organ, whether attached to the original body, detached and being preserved or prepared for transplant, or attached to a new body. During these circulations and perfusions, aggregated platelets tend to block blood vessels and portions of the circulation apparatus. This blocking may be avoided by the presence of the active compounds. For this purpose, the combination may be added gradually or in

single or multiple portions to the circulating blood, to the blood of the donor animal, to the perfused body portion, detached or attached, to the recipient, or to two or all of these at a total steady dose of from 0.001 to 10 mg per litre of circulating fluid. It is useful to use the combinations in laboratory animals, e.g. cats, dogs, rabbits, monkeys, and rats, for these purposes to develop new methods and techniques for organ or limb transplant.

An active compound, such as 5-(6-carboxy-hexyl)-1-(3-hydroxyoctyl)-hydantoin, 5-(6-carboxyhexyl)-3-methyl-1-(3-oxo-octyl)hydantoin, 5-(6-carboxyhexyl)-1-(3-oxo-octyl)hydantoin or 5-(6-carboxyhexyl)-1-(4-phenoxybutyl) hydantoin, which inhibits pentagastrin-induced gastric acid secretion and reduces the formation of aspirin-induced gastric lesions in rats may be useful in a combination in reducing excessive gastric acid secretion, reducing and avoiding gastro-intestinal ulcer formation and accelerating the healing of such ulcers already present in the gastrointestinal tract whether such ulcers arise spontaneously or as a component of polyglandular adenoma syndromes.

Intravenous infusions of certain combinations, typically those wherein 5-(6-carboxyhexyl)-1-(3-hydroxyoctyl) hydantoin is present, may have a potential utility as diuretic agents, the uses of which include the treatment of oedema, for example oedema associated

with heart failure, liver failure or kidney failure in man or other mammals.

A further use for combinations containing an active compound which has an effect on the uterine smooth muscle is as anti-fertility agents, in particular as abortifacients; for example, in controlling conception.

A combination comprising an active compound which antagonises histamine-induced broncho-constriction, such as 5-(6-carboxyhexyl)-1-(3-hydroxy-4,4-dimethyloctyl) hydantoin, and a phosphodiesterase inhibitor may be used in the treatment or prophylaxis of bronchial asthma and bronchitis by alleviating the broncho-constriction associated with this condition.

The combinations of the present invention may also be used in the treatment of proliferative skin diseases, such as psoriasis, atopic dermatitis, non-specific dermatitis, primary irritant contact dermatitis, allergic contact dermatitis, basal and squamous cell carcinomas of the skin, lamellar ichthyosis, epidermolytic hyperkeratosis, pre-malignant sun-induced keratosis, non-malignant keratosis, acne and seborrheic dermatitis in humans and atopic dermatitis and mange in domestic animals.

The amount of active compound present in a combination required for therapeutic effect will vary not only with the particular compound and the route of administration but also with the particular phoshodiesterase inhibitor used and its amount, i.e. with the degree of potentiation that

occurs. The amount of active compound, will, however, be below that required to produce a given therapeutic effect in the absence of the potentiator and thus also below the level which would produce a significant vasodilatory effect.

The amount of an active compound required in the absence of a phosphodiesterase inhibitor to achieve the desired pharmacological effect will of course depend on a number of factors; for example, the active compound chosen, the use for which it is intended, the mode of administration, and the recipient. In general, a daily dose lies in the range of from 1 µg to 20 mg per kilogram bodyweight. Preferably the daily dose is 10 µg to 2 mg, especially 100 µg to 0.2 mg (200 µg), per kilogram bodyweight. For example, an intravenous dose may lie in the range of from 5 µg to 1 mg/kg, preferably 50 µg to 100 µg/kg which may conveniently be administered as an infusion of from 0.01 to 50 µg preferably 0.1 to 5 µg, especially 0.5 to 1.5 µg, per kilogram per minute. Infusion fluids suitable for this purpose may contain from 0.001 to 100 µg/ml, for example from 0.01 to 10 µg per millilitre. Unit doses may contain from 10 µg to 100 mg of an active compound depending on how the compound is to be administered for example ampoules for injection may contain from 0.01 to 1 mg, preferably 0.05-0.15 mg, for example 0.1 mg; and orally administrable unit dose formulations such as tablets or capsules may contain from 0.1 to 50, preferably 2 to 20, mg, especially 5 to 15 mg, for example 10 mg.

JDM/LM/18 October 1979

More specifically, when an active compound is used in the absence of a phosphodiesterase inhibitor to inhibit platelet aggregation it is generally desirable to achieve a concentration in the appropriate liquid, whether it be the blood of a patient or a perfusion fluid, of about 1 µg to 10 mg per litre, preferably from 10 µg to 1 mg per litre, especially 0.05 to 0.15 mg per litre, for example 0.1 mg per litre.

However, in the present invention where a phosphodiesterase inhibitor is used a suitable dose of active compound will in general be about half or, say, up to about 75% of the dose required in the absence of phosphodiesterase inhibitor, e.g. 0.05 to 25, e.g. 2.5 to 7.5 mg e.g. 5 mg, for tablets.

In general the active compound and phosphodiesterase inhibitor are administered in a weight ratio of 1 part of active compound to from 1 to 200, preferably from 10 to 150, especially 40 to 100, e.g. 60, parts of phosphodiesterase inhibitor.

While it is possible for an active compound and a phosphodiesterase inhibitor to be administered as raw chemicals, it is preferable to present them as one or more pharmaceutical formulations.

A unit dose of a formulation of a combination may contain from 0.05 to 25 mg, preferably 1 to 10 mg, especially 2.5 to 7.5 mg e.g. 5 mg of the active compound,

and from 100 to 400 mg, e.g. from 250 to 350 mg, e.g. 300 mg of the phosphodiesterase inhibitor. Accordingly a unit dose formulation may contain for example, 300 mg of phosphodiesterase inhibitor and 5 mg of an active compound.

The abovementioned doses of the active compounds refer to the acids, amides, esters, alcohols and tetrazoles etc of the active compounds; where a salt is used, the dose should be taken as referring to the corresponding anion.

A formulation of the present invention both for veterinary and for human medical use, comprises a combination together with one or more acceptable carriers therefor and optionally other therapeutic ingredient(s). When the active compound and phosphodiesterase inhibitor are to be administerd separately, the formulation to be used comprises the active compound together with one or more acceptable carriers therefor and optionally other therapeutic ingredient(s); the phosphodiesterase inhibitor formulation may comprise any formulation in which such a compound is normally presented. The carrier must be 'acceptable' in the sense of being compatible with the other ingredients of the formulation and not deleterious to the recipient therefor.

Formulations include those suitable for oral e.g. buccal, rectal, vaginal or parenteral (including subcutaneous, intramuscular or intravenous injection or infusion) or intrapulmonary administration, although the most suitable route in a given case will depend upon the active compound.

The formulations may conveniently be presented in unit dosage form and may be prepared by methods known in the art of pharmacy. All such methods include the step of bringing into association the combination with the carrier which constitutes one or more accessory ingredients. In general the formulations are prepared by uniformly and intimately bringing into association, e.g. by admixing, the combination with a liquid carrier or a finely divided solid carrier or both, and then, if necessary, shaping the formulation into the desired presentation.

Formulations according to the invention suitable for oral administration may be presented as discrete units such as a capsule, cachet, lozenge or tablet each containing a predetermined amount of the combination as a powder or granules; or as a solution or a suspension in an aqueous or non-aqueous liquid; or as an oil-in-water emulsion or a water-in-oil liquid emulsion.

A formulation according to the invention for parenteral administration may be presented in an ampoule for receiving a defined quantity of liquid for making a solution for infusion.

A tablet may be made by compression or moulding, optionally with one or more accessory ingredients. Compressed tablets may be prepared by compressing in a suitable machine the combination in a free-flowing form, such as a powder or granules, optionally mixed with a binder, lubricant, pharmaceutically inert diluent, surface active or dispersing agent. Moulded tablets may be made by moulding in a suitable machine a combination together with a suitable carrier moistened with a pharmaceutically inert liquid diluent.

Formulations for rectal administration may be presented as a suppository with a conventional carrier, such as cocoa butter.

Formulations suitable for vaginal administration may be presented as a pessary, cream, paste or spray formulation containing, in addition to the combination, such carriers as are known in the art to be appropriate.

Formulations suitable for parenteral administration conveniently comprise a sterile aqueous preparation of the combination which is preferably isotonic with the blood of the recipient.

As the active compound can be absorbed through the skin into the blood of the recipient, it may be administered topically in a topical formulation. The phosphodiesterase inhibitor may be administered by another route, unless, of course, it can be absorbed

through the skin into the blood when in which case it can be administered topically. For the treatment of wounds in or close to the skin topical administration of the formulation, with or without phosphodiesterase inhibitor, is particularly appropriate.

For example, a formulation of the combination may be presented as a cream or lotion, or a formulation of the active compound may be presented topically as a cream or lotion and the phosphodiesterase inhibitor may be administered orally or by any other suitable route.

It should be understood that, in addition to the aforementioned ingredients, the formulations of the invention may include one or more additional ingredients, such as a diluent, buffer, lubricant, or preservative (including an anti-oxidant).

It will be appreciated from the foregoing that what we shall claim may comprise any novel feature described herein, for example:

(a) a combination comprising an active compound as hereinbefore defined together with a phosphodiesterase inhibitor;

(b) a formulation comprising a combination as defined in (a) in association with a pharmaceutically acceptable carrier therefor;

(c) a method for the preparation of such a combination or formulation;

JDM/LM/18 October 1979

(d) a method of promoting the inhibition of platelet aggregation which comprises the bringing of said platelets into association with an effective platelet aggregatory inhibiting amount of an active compound and a phosphodiesterase inhibitor;

(e) a method for the treatment or prophylaxis of thrombosis in a mammal or mammalian tissue, including human, which comprises the administration to said mammal of a non-toxic, effective anti-thrombotic amount of an active compound and a phosphodiesterase inhibitor;

(f) a method for the treatment or prophylaxis of thrombo-embolic disorders in a mammal, including man, which comprises the administration to said mammal of a non-toxic, effective anti-thrombic amount of an active compound and a phosphodiesterase inhibitor;

(g) a method for the treatment or prophylaxis of a gastric lesion in a mammal, including man, comprising the administration to said mammal of a non-toxic, effective prophylactic or therapeutic amount of an active compound and a phosphodiesterase inhibitor;

(h) a method for inducing bronchodilation in a mammal, including man, comprising the administration to said mammal of a non-toxic, effective bronchodilatory amount of an active compound and a phosphodiesterase inhibitor;

(i) a method for the treatment or prophylaxis of an allergic condition in a mammal, including man, comprising the administration to said mammal of a non-toxic, effective prophylactic or therapeutic amount of an active compound and a phosphodiesterase inhibitor;

(j) a method of inducing abortion of a foetus in a mammal, including man, comprising the administration to said mammal of a non-toxic, effective abortifacient amount of an active compound, and a phosphodiesterase inhibitor;

(k) a method of controlling conception in a mammal, includi man, comprising the administration to said mammal of a non-toxic, effective contraceptive amount of an active compound and a phosphodiesterase inhibitor;

(l) a combination, formulation or method of treating a mammal according to any of (a) to (k) wherein the active compound is selected from the class consisting of 1,5-disubstituted hydantoin and thiohydantoin derivatives optionally further substituted in the 3-position and/or the 5-position and their salts;

(m) a combination, formulation or method of treating a mammal according to any of (a) to (l) wherein the active compound is selected from the class consisting of compounds of formula (I) as hereinbefore defined;

(n) a combination, formulation or method of treating a mammal according to any of (a) to (m) wherein the active compound is 5-(6-carboxyhexyl)-1-(3-hydroxy-3-cyclohexylpropyl) hydantoin or 5-(6-carboxyhex-2-enyl)-1-(3-hydroxy-3-cyclohexylpropyl) hydantoin;

(o) a combination, formulation or method of treating a mammal according to any of (a) to (n) wherein the phosphodiesterase inhibitor is selected from the class

consisting of derivatives of either xanthine or pyrimido (5,4-d) pyrimidine as hereinbefore described; and

(p) a combination, formulation or method of treating a mammal according to any of (a) to (o) wherein the phosphodiesterase inhibitor is theophylline or dipyridamole.

The following Examples illustrate the present invention

JDM/LM/18 October 1979

Where the active compound and the phosphodiesterase inhibitor are administered separately, the active compound may be administered using the following formulations. The phosphodiesterase inhibitor, e.g. dipyridamole, may be administered using formulations in which it is available to give a dose of about 300 mg.

Reference Example 1

| Tablet | In one tablet |
| --- | --- |
| 5-(6-Carboxyhexyl)-1-(3-hydroxy-3-cyclohexylpropyl) hydantoin | 5.0 mg |
| Lactose B.P. | 82.0 mg |
| Starch B.P. | 10.0 mg |
| Povidone B.P.C. | 2.0 mg |
| Magnesium Stearate | 1.0 mg |

Mix together the active compound, lactose and starch. Granulate the powders using a solution of the povidone in purified water. Dry the granules, add the magnesium stearate and compress to produce tablets, 100 mg per tablet.

Reference Example 2

| Capsule | In one capsule |
| --- | --- |
| 5-(6-Carboxyhexyl)-1-(3-hydroxy-3-cyclohexylpropyl) hydantoin (m.p. 96-98$^{\circ}$) | 5 mg |
| Lactose | 84 mg |
| Starch | 10 mg |
| Magnesium Stearate | 1 mg |

Mix the powdered ingredients in a powder blender, fill into hard gelatin capsules, 100 mg per capsule.

JDM/LM/18 October 1979

Reference Example 3

1 µg/ml Injection

| | | |
|---|---|---|
| 5-(6-Carboxyhexyl)-1-(3-hydroxy-3-cyclohexylpropyl)hydantoin (m.p. 96-98°) | | 100 µg |
| Water for Injections | q.s. to...... | 100 ml |

Dissolve the active compound in the Water for Injections. Sterilise the solution by filtration through a membrane filter, 0.22 µm pore size, collecting the filtrate in a sterile receiver. Under aseptic conditions, fill the solution into sterile glass ampoules, 1 ml per ampoule. Seal by fusion of the glass.

Reference Example 4

10 µg/ml Injection

| | | |
|---|---|---|
| 5-(6-Carboxyhexyl)-1-(3-hydroxy-3-cyclohexylpropyl)hydantoin (m.p. 96-98°) | | 1 mg |
| Ethyl Alcohol | | 10 mg |
| Propylene Glycol | | 30 ml |
| Water for Injections | q.s. to.... | 100 ml |

Dissolve the active compound in the ethyl alcohol, add the propylene glycol and dilute to volume with Water for Injections.

Sterilise the solution by filtration through a membrane filter, 0.22 µm pore size, collecting the filtrate in a sterile vessel. Under aseptic conditions, fill the solution into sterile glass vials, 10 ml per vial. Close with a sterile rubber plug and secure with an aluminium collar.

JDM/LM/16 October 1979

Reference Example 5

100 µg Single dose injection (freeze-dried)

| | |
|---|---|
| 5-(6-Carboxyhexyl)-1-(3-hydroxy--3-cyclohexylpropyl)hydantoin (m.p. 96-98°) | 10.0 mg |
| Mannitol | 2.5 g |
| N/10 Sodium Hydroxide Solution q.s. to.... | pH 10.0 |
| Water for Injections q.s. to..... | 100.0 ml |

Suspend the active compound in approximately 20 ml Water for Injections. Add sufficient sodium hydroxide solution to produce pH 10 and stir to dissolve the active compound. Add and dissolve the mannitol and dilute to volume with Water for Injections.

Sterilise the solution by passage through a membrane filter, 0.22 µm pore size and distribute aseptically into sterile vials, 1 ml per vial. Freeze dry the solutions and seal the containers under aseptic conditions with rubber closures. Each vial contains 100 µg active compound as its freeze-dried sodium salt.

Reference Example 6

Suppository

| | |
|---|---|
| 5-(6-Carboxyhexyl)-1-(3-hydroxy-3-cyclohexylpropyl)hydantoin (m.p. 96-98°) | 3 mg |
| Massa Esterinum C q.s. to...... | 2 g |

Melt the suppository base at around 40°C. Gradually incorporate the active compound in fine powder and mix until homogeneous. Pour into suitable moulds and allow to set.

JDM/LM/16 October 1979

Massa Esterinum C is a commercially available suppository base consisting of a mixture of mono-, di- and tri-glycerides of saturated vegetable fatty acids. It is marked by Henkel International, Dusseldorf.

When the active compound and phosphodiesterase inhibitor are to be administered together, the following formulations may be used.

EXAMPLE 7

| Tablet | In one tablet |
|---|---|
| 5-(6-Carboxyhexyl)-1-(3-hydroxy-3-cyclohexylpropyl)hydantoin (m.p. 96-98°) | 5.0 mg |
| Dipyridamole | 300.0 mg |
| Starch B.P. | 30.0 mg |
| Povidone B.P.C. | 3.0 mg |
| Magnesium Stearate | 2.0 mg |

Mix together the active compound, dipyridamole and starch. Granulate the powders using a solution of the povidone in purified water. Dry the granules, add the magnesium stearate and compress to produce tablets, 340 mg per tablet.

EXAMPLE 8

| Capsule | In one capsule |
|---|---|
| 5-(6-Carboxyhexyl)-1-(3-hydroxy-3-cyclohexylpropyl)-hydantoin (m.p. 96-98°) | 5 mg |
| Dipyridamole | 300 mg |
| Starch | 30 mg |
| Magnesium Stearate | 2 mg |

0011828

Mix the powdered ingredients in a powder blender, fill into hard gelatin capsules, 337 mg per capsule.

EXAMPLE 9

Suppository

| | | |
|---|---|---|
| 5-(6-Carboxyhexyl)-1-(3-hydroxy-3-cyclohexylpropyl)hydantoin (m.p. 96-98°) | | 5 mg |
| Dipyridamole | | 50 mg |
| Massa Esterinum C | q.s. to...... | 2 g |

Melt the suppository base at around 40°C. Gradually incorporate the active compound and dipyridamole in fine powder and mix until homogeneous. Pour into suitable moulds and allow to set.

EXAMPLE 10

2 μg/ml Injection

| | | |
|---|---|---|
| 5-(6-Carboxyhexyl)-1-(3-hydroxy-3-cyclohexylpropyl)hydantoin (m.p. 96-98°) | | 0.2 mg |
| Dipyridamole | | 0.2 g |
| Ethyl Alcohol | | 10 ml |
| Propylene Glycol | | 30 ml |
| Water for Injections | q.s. to ....... | 100 ml |

Dissolve the active compound and dipyridamole in the ethyl alcohol, add the propylene glycol and dilute to volume with Water for Injections.

Sterilise the solution by filtration through a membrane filter, 0.22 μm pore size, collecting the filtrate in a sterile vessel. Under aseptic conditions, fill the solution into sterile neutral glass ampoules 5 ml per ampoule.

JDM/LM/16 October 1979

The ampoule may be used as a 5 ml infusion for slow intravenous administration.

Where the active compound and phosphodiesterase inhibitor are to be administered together, the following combination may be used.

EXAMPLE 11

| Combination | In one dose |
| --- | --- |
| 5-(6-Carboxyhexyl)-1-(3-hydroxy-3-cyclohexyl-propyl)hydantoin | 7 mg |
| Dipyridamole | 420 mg |

The combination may be administered after mixing together the powdered active compound and dipyridamole.

EXAMPLE 12

Biological results

These were obtained using 5-(6-carboxyhexyl)-1-(3-hydroxy-3-cyclohexylpropyl)hydantoin and dipyridamole in combination.

Aggregation was induced in rabbit platelet-rich plasma (PRP) by addition of arachidonic acid, and monitored in a Born-type aggregometer. A dose of dipyridamole was selected which caused no inhibition of aggregation when added to the PRP 2 minutes prior to the addition of arachidonic acid. The active compound when added to the PRP 1 minute prior to the addition of arachidonic acid caused a dose-related inhibition of aggregation. The percentage inhibition caused by the active compound was compared in the presence and absence of the selected dose of dipyridamole.

The results are set out in Table A.

TABLE A

| Dipyridamole (μg/ml) | Inhibitor 5-(6-carboxyhexyl)-1-(3-hydroxy-3-cyclohexylpropyl) hydantoin ng/ml | Percentage Inhibition |
|---|---|---|
| 50 | - | 0 |
| - | 50 | 37 |
| 50 | 50 | 77 |
| - | 75 | 66 |
| 50 | 75 | 100 |
| - | 100 | 100 |

Therefore, dipyridamole potentiates the inhibitory activity of the active compound on platelet aggregation, so that the inhibitory effect of a dose of the active compound in the presence of dipyridamole is approximately equivalent to that caused by twice that dose of the active compound alone.

EXAMPLE 13

Since 5-(6-carboxyhexyl)-1-(3-hydroxy-3-cyclohexylpropyl)hydantoin is not a particularly potent inhibitor of rabbit platelet aggregation the experiment of Example 11 was repeated using human PRP and a similar potentiation was observed with dipyridamole.

JDM/LM/19 October 1979

EXAMPLE 14

The experiment of Example 12 was repeated using theophylline, another inhibitor of phosphodiesterase, in place of the dipyridamole. Theophylline was also found to potentiate the inhibitory effect of 5-(6-carboxyhexyl)-1-(3-hydroxy-3-cyclohexylpropyl)hydantoin on ADP - induced aggregation of human PRP. The results are set out in Table B.

TABLE B

| Theophylline (µg/ml) | Inhibitor 5-(6-carboxyhexyl)-1-(3-hydroxy-3-cyclohexylpropyl)hydantoin ng/ml | Percentage Inhibition |
|---|---|---|
| 20 | - | 7 |
| - | 0.5 | 19 |
| 20 | 0.5 | 51 |
| - | 1 | 55 |
| 20 | 1 | 70 |

JDM/LM/19 October 1979

0011828

CLAIMS:

A590 Eur

1. A pharmaceutical combination which comprises:

(1) an active compound which is a derivative of either hydantoin or thiohydantoin, or a salt of either of these and

(2) a cyclic AMP phosphodiesterase inhibitor

2. A combination as claimed in claim 1 wherein the active compound is a 1,5-disubstituted hydantoin or 1,5-disubstituted thiohydantoin derivative either of which may be optionally further substituted in the 3- and/or 5-position, or a salt of either of them.

3. A combination as claimed in claim 2 wherein the active compound is of the formula (I)

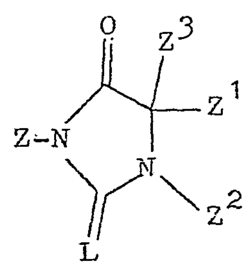

(I)

wherein

L is O or S;

Z and $Z^3$ are the same or different and each is hydrogen or alkyl of 1 to 6 carbon atoms;

one of $Z^1$ and $Z^2$ is a group $-CH_2-X-X^1-X^2$ wherein

X is phenylene, $-C{\equiv}C-$, cis or trans $-CH{=}CH-$ or $-CH_2-CQ_2-$ in which each Q may be the same or different and is hydrogen or alkyl or the two Q's together form an alkylene radical of four, five or six carbon atoms;

$X^1$ is a covalent bond or a straight or branched alkylene chain having 1 to 6 carbon atoms optionally having one of its methylene groups replaced by oxa (-O-) or thia (-S-) provided that at least one carbon atom separates any oxa or thia from a -C≡C-, -CH=CH- or -CO- group; and

$X^2$ is 5-tetrazolyl, carboxyl, carboxamide, hydroxymethylene or alkoxycarbonyl;

and the other of $Z^1$ and $Z^2$ is a group $-Y-Y^1-Y^2-Y^3$ wherein

Y is $-CR_2-CH_2-$ in which each R may be the same or different and is hydrogen or methyl;

$Y^1$ is carbonyl, methylene, methylene substituted by hydroxyl or methylene substituted by hydroxyl and alkyl;

$Y^2$ is a covalent bond or straight or branched alkylene having 1 to 7 carbon atoms optionally substituted in the carbon adjacent $Y^1$ by one or two groups each of which may be alkyl or a cyclic radical;

$Y^3$ is hydrogen, hydroxy, alkoxy of 1 to 7, preferably 1 to 4, carbon atoms, a cyclic radical, phenyl, benzyl, phenoxy or benzyloxy, wherein each of phenyl, benzyl, phenoxy and benzyloxy may be substituted in the benzene ring by one or more groups selected from hydroxy, halogeno, nitro, amino, acylamino, alkenyl, alkoxy, phenyl and alkyl which may itself be substituted by one or more halogeno groups; or $Y^2$ and $Y^3$ together form an alkyl group of 1 to 7 carbon atoms having at least one hydrogen replaced by fluoro;

or Y is a bond, $-CH_2-$ or $-CH_2.CH_2-$ and $Y^1$, $Y^2$ and $Y^3$ taken together form a cycloalkyl or bicyclocalkyl group substituted by a hydroxyl group which preferably has three carbon atoms separating it from the hydantoin ring; and their salts;

and said term 'cyclic radical' means a monovalent radical derived by loss of a ring hydrogen atom from a monocyclic or polycyclic compound having from 3 to 12 ring atoms selected from carbon, nitrogen, oxygen and sulphur, which compound may be saturated or unsaturated and may be further substituted by one or more alkyl groups, but excluding phenyl.

4. A combination as claimed in claim 3 wherein the active compound is 5-(6-carboxyhexyl)-1-(3-hydroxy-3-cyclohexylpropyl)hydantoin or 5-(6-carboxyhex-2-enyl)-1-(3-hydroxy-3-cyclohexylpropyl)hydantoin.

5. A combination as claimed in any of the preceding claims wherein the phosphodiesterase inhibitor is a derivative of xanthine or a derivative of pyrimido (5,4-d)pyrimidine.

6. A combination as claimed in claim 6 wherein the phosphodiesterase inhibitor is theophylline or dipyridamole.

7. A combination as claimed in any of the preceding claims which comprises 5-(6-carboxyhexyl)-1-(3-hydroxy-3-cyclohexylpropyl)hydantoin or 5-(6-carboxyhex-2-enyl)-1-(3-hydroxy-3-cyclohexylpropyl)hydantoin together with theophylline or dipyridamole.

8. A method of preparing a combination as defined in any of the preceding claims which comprises admixing the

active compound and the phosphodiesterase inhibitor.

9. A pharmaceutical formulation which comprises:

(1) an active compound which is a derivative of either hydantoin or thiohydantoin, or a salt of either of these and

(2) a cyclic AMP phosphodiesterase inhibitor together with

(3) a pharmaceutically acceptable carrier therefor.

10. A formulation as claimed in claim 9 wherein the carrier is a solid.

11. A formulation as claimed in claim 9 wherein the carrier is a liquid.

12. A formulation as claimed in any of claims 9 to 11 in a form suitable for oral, parenteral, rectal, vaginal or intrapulmonary administration.

13. A formulation as claimed in any of claims 9 to 12 in unit dosage form.

14. A formulation as claimed in claim 13 in tablet form.

15. A formulation as claimed in any of claims 9 to 14 containing from 0.05 to 25 mg of the active compound.

16. A formulation as claimed in claim 15 containing from 100 to 400 mg of the phosphodiesterase inhibitor.

17. A method for preparing a formulation as defined in any of claims 9 to 16 which comprises admixing the combination as defined in ay of claims 2 to 7 with the pharmaceutically acceptable carrier therefor.

JDM/LM/26 October 1979

18. A combination or formulation as claimed in any of the preceding claims for use in potentiating the inhibition of platelet aggregation.

19. A combination or formulation as claimed in claim 18 for use in the treatment or prophylaxis of thrombo-embolic disorders in a mammal.

20. A combination or formulation as claimed in either of claims 18 or 19 for use in the treatment or prophylaxis of thrombosis in a mammal.

21. A combination or formulation as claimed in any of claims 1 to 17 for use in the treatment or prophylaxis of a gastric lesion in a mammal.

22. A combination or formulation as claimed in any of claims 1 to 17 for use in the induction of bronchodilation in a mammal.

23. A combination or formulation as claimed in any of claims 1 to 17 for use in the treatment or prophylaxis of an allergic condition in a mammal.

24. A combination or formulation as claimed in any of claims 1 to 17 for use in the induction of abortion of a foetus in a mammal.

25. A combination or formulation as claimed in any of claims 1 to 17 for use in the control of conception in a mammal.

26. A combination or formulation as claimed in any of claims 1 to 17 for use in any of the conditions as defined in any of claims 18 to 25 which comprises a dose of the

active compound in the range of from 1 µg to 20 mg/kg/day and a dose of the phosphodiesterase inhibitor in a weight ratio of 1 part of the active compound to from 1 to 200 parts of the phosphodiesterase inhibitor.

JDM/LM/26 October 1979

0011828

European Patent Office

# EUROPEAN SEARCH REPORT

Application number
EP 79 10 4650

## DOCUMENTS CONSIDERED TO BE RELEVANT

| Category | Citation of document with indication, where appropriate, of relevant passages | Relevant to claim |
| --- | --- | --- |
| X | ROTE LISTE, 1963<br>Editio Cantor<br>Aulendorf/Württ<br>Page 1212, "Zentromid"<br>-- | 1,5,8-26 |
| X | GB - A - 1 043 842 (SANDOZ PATENTS LIMITED)<br>* Page 1, lines 9-29; page 4, claims 1-3 *<br>-- | 1,5,8-26 |
| X | FR - A - 2 380 779 (MICHAELI Dov)<br>* Pages 7,8; example 2; page 10, claim 7 *<br>-- | 1,8-26 |
| | NL - A - 78 05402 (THE WELLCOME FOUNDATION LIMITED)<br>* Page 2, line 32 - page 3, line 31; pages 17-18; claims 1-8 *<br>& FR - A - 2 390 964<br>-- | 1-26 |
| | FR - A - 2 235 678 (MERCK & CO. INC.)<br>* Page 3, lines 18-23; pages 10-11; claims 1-14 *<br>-- | 1-26 |
| | US - A - 4 051 236 (E.R. SQUIBB & SONS, INC.)<br>* Claims 1-7 *<br>-- | 1-26 |
| A | FR - A - 2 362 839 (THE WELLCOME FOUNDATION LIMITED)<br>./. | 1-26 |

**CLASSIFICATION OF THE APPLICATION (Int Cl.)**

A 61 K 45/06
31/52
31/505//
(A 61 K 31/52
31/415)
(A 61 K 31/505
31/415)

**TECHNICAL FIELDS SEARCHED (Int.Cl.)**

A 61 K 31/505
31/52
31/485
31/535
31/55
31/44
31/445
31/415

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
| --- | --- | --- |
| The Hague | 10-03-1980 | BRINKMANN |

EPO Form 1503.1 06.78

European Patent Office

# EUROPEAN SEARCH REPORT



| Category | Citation of document with indication, where appropriate, of relevant passages | Relevant to claim | CLASSIFICATION OF THE APPLICATION (Int. Cl. 3) |
|---|---|---|---|
| | * Page 12, line 30 - page 13, line 33; page 30, lines 1-2; page 40, example 44; pages 47-49; claims 1-9 * | | |
| D | & BE - A - 855 337 | | |
| | -- | | |
| A | FR - A - 2 374 309 (BEECHAM GROUP LIMITED) | 1-26 | |
| | * Page 12, line 34 - page 13, line 6; pages 49-50; claims 1-11 * | | TECHNICAL FIELDS SEARCHED (Int. Cl. 3) |
| D | & BE - A - 861 956 | | |
| | -- | | |
| P,D A | EP - A - 0 003 410 (BEECHAM GROUP LIMITED) | 1-26 | |
| | * Page 14, line 26 - page 15, line 6; pages 38,39; claims 1-12 * | | |
| | -- | | |
| P,A | EP - A - 0 002 258 (THE WELLCOME FOUNDATION LIMITED) | 1-26 | |
| | * Page 1, lines 5-14; claims 1-12 * | | |
| | -- | | |
| P,A | EP - A - 0 002 259 (THE WELLCOME FOUNDATION LIMITED) | 1-26 | |
| | * Page 1, lines 5-14; claims 1-11 * | | |
| | ---- | | |